# EUROPEAN PATENT SPECIFICATION

(11) **EP 0 765 648 B1**
(45) Date of publication and mention of the grant of the patent: **14.01.2004**
(21) Application number: 96306887.9
(22) Date of filing: 20.09.1996
(51) Int. Cl.: A61F 9/00

(54) **Ophthalmic surgery apparatus**
Gerät für die Augenchirurgie
Appareil de chirurgie ophtalmique

(30) Priority: 29.09.1995 JP 27632795; 06.08.1996 JP 22450996
(43) Date of publication of application: 02.04.1997
(73) Proprietor: NIDEK CO., LTD, Gamagori-shi, Aichi 443-0035 (JP)
(72) Inventor: Sumiya, Toshihumi, Kouta-cho Nukata-gun, Aichi 444-0116 (JP); Kanda, Hidenori, Okazaki-shi Aichi 443-2134 (JP); Arashima, Mikio, Takeya-cho, Gamagori-shi Aichi, 443-0046 (JP)
(74) Representative: Blatchford, William Michael

(56) References cited:
- WO-A-93/16631
- WO-A-94/18883
- DE-A- 4 232 021
- US-A- 4 443 075
- US-A- 4 848 340
- US-A- 5 098 426

## Description

The present invention relates to an ophthalmic surgery apparatus for treatment by irradiating a laser beam for treatment to an eye of a patient, and more particularly, it relates to a mechanism for tracking a movement of an eye of a patient and for guiding a laser beam for treatment to the desired position.

As an ophthalmic surgery apparatus for treatment by irradiating a laser beam for treatment to an eye of a patient, for example, a cornea surgery apparatus which uses an excimer laser beam is known. This apparatus resects inflammation of surface layer of a cornea by irradiating an excimer laser beam to the surface of the cornea, and corrects a refractive ametropia by removing the surface of the cornea and by changing the curvature of the cornea.

This apparatus fixes the fixation target to the eye of the patient and the ophthalmologist aligns an irradiating optical system with the eye of the patient so as to be desired conditions, with his eyes observing the alignment target. The alignment has been completed, and then, a desired volume of a desired region is ablated by controlling the apparatus.

The ophthalmologist fixes the fixation target to the eye of the patient and he fixes the eye of the patient, but in the case that the patient of which fixation is bad, his eyeball happens to move, and then the movement is confirmed by the ophthalmologist, then he must do over again once more from beginning of the alignment, or he must discontinue the laser beam irradiation and must do over again once more from the alignment.

As described above, doing the alignment over again once more causes taking much a long time for its operation or the like, and then it is burden to both the patient and the ophthalmologist. Furthermore, in the case that the eyeball of the patient moves often, the burden is much more.

And if the ophthalmologist does not notice the movement of the eyeball and leaves the eyeball as it is, and then he continues the laser beam irradiation with the eyeball moving, the cornea has not been removed so as to be the shape expected, therefore the refractive power of the eye after the operation is influenced thereby.

Furthermore, if the ophthalmologist is not accustomed to operating machine, the alignment itself takes much time.

WO 94/18883 discloses apparatus and system for ophthalmic surgery, in which a tracking system includes means for (a) sensing contrast in recognizable large scale boundaries such as the change between the cornea/sclera interface (limbus), thereby to determine the absolute location and orientation of these boundaries; and (b) dual mode operation of an electronic control system compatible with all analog technologies, substantially increasing the speed of operations over other methods. The tracking system comprises illumination sources imaging optics, a sensor such as a position-sensitive detector, a movable optical element such as mirror, a two-dimensional logic board and a dedicated microprocessor, including apparopriate signal processing firmware and software. Additional optics can be incorporated to provide interface with other assemblies such as depth tracking, target viewing and/or laser surgery sub-systems.

US-A-4 848 340 discloses an eyetracker for moving the path of a laser beam in response to patient eye movement comprises a visual light source on which the patient can fixate to bring the visual axis of the eye into a reference alignment with the optical axis of the light source. A laser source, having its beam coaxially aligned with the optical axis of the light source, cuts a mark onto the cornea of the eye when the eye is in the reference alignment. An infrared optical system monitors movement of the mark to generate an error signal which is proportional to any movement of the mark out of the reference alignment. Means are also provided by the eyetracker to move the path of the laser beam back into the reference alignment to reduce the error signal to a null.

DE-A-42 32 021 discloses an apparatus for treating the cornea of an eye by ablation with a laser beam, in which during the treatment a first position of the eye is fixed and is compared with a required position in agreement with the optical axis of the eye. The laser beam treatment is switched off upon deviation of the position of the eye to be treated from the required position.

The present invention has been made in view of the above circumstances and has an object to overcome the above problems, and to provide an ophthalmic surgery apparatus which may not burden both a patient and an ophthalmologist, can make it easy to align with the laser beam irradiating optical system, and can achieve appropriate treatment.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

In accordance with the present invention, there is provided an ophthalmic surgery apparatus comprising:
an observation optical system for observing an anterior portion of an eye of a patient; and
a laser beam irradiating optical system for producing an irradiating laser beam for causing ablation of a cornea of the eye,
means for aligning said irradiating optical system with the eye
said ophthalmic surgery apparatus being adapted to ablate the cornea of an area to be used for sight of the eye under the condition that said irradiating optical system is aligned with the eye, the apparatus comprising:
   moving means for moving an irradiating position of the laser beam by said irradiating optical system;
   illuminating means for illuminating an anterior portion of the eye of the patient including an iris of the eye;
   photoelectric transducing means having a two-dimensional photographing element for photographing a pupil and the iris of the eye illuminated by said illuminating means;
characterized in that the apparatus comprises:
pupil center position detecting means for detecting the position of a pupil center based on pupil information obtained by processing a signal transmitted from said photographing element;
storage means for storing the pupil center position detected by said pupil center position detecting means when alignment before surgery is completed or a predetermined position of said photographing element; and
control means for controlling said moving means by comparing the detected pupil center position and the stored position and obtaining deviation between the detected pupil center position and the stored position so that the detected pupil center position may be placed within a predetermined permissible range of the stored position.

According to the present invention, it is capable of prohibiting an irradiating region from shifting, and is capable of treating appropriately an eye of a patient, because the apparatus can track the eye of the patient even if the eyeball moves during the alignment or during the operation by using the laser irradiating treatment. Also, since the performance for operating is improved, so the burden is allowed to be decreased for both the patient and the ophthalmologist. Furthermore, the trouble of the alignment is allowed to be decreased, therefore the alignment adjustment can be performed easily.

The invention will now be described by way of example with reference to the accompanying drawings in which:
Fig. 1 is an external view showing a whole schematic configuration of an apparatus for a corneal surgery according to the preferred embodiment of the present invention;
Fig. 2(a) is a view showing the optical elements arrangement of the inside of the arm part of the apparatus;
Fig. 2(b) is a view showing mechanism of movement of an arm part;
Fig. 3 is a schematic view showing the optical system of the apparatus of the preferred embodiment of the present invention;
Fig. 4 is a view showing an anterior portion of an eye of a patient photographed by a CCD camera;
Fig. 5 is a view showing distribution of light volume on line A-A' shown in Fig. 4;
Fig. 6 is a block diagram for a control system of important part of the apparatus according to the preferred embodiment of the present invention;
Fig. 7 is a view showing the example for the image divided into 16 areas, which centers optical axis O against the two-dimensional image sent by CCD camera; and,
Fig. 8 is a flowchart illustrating the process of the automatic alignment which moves the arm part to XY-direction based on the value of the light-and-shade information.

One preferred embodiment according to the present invention will be now described with reference to the accompanying drawings.

### General Construction

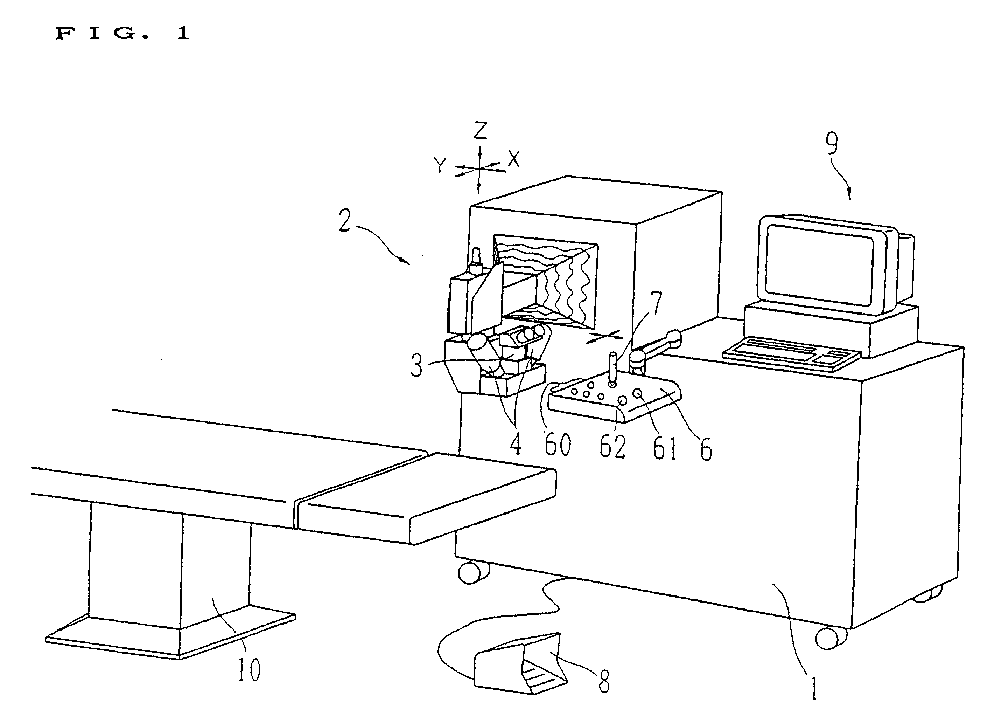

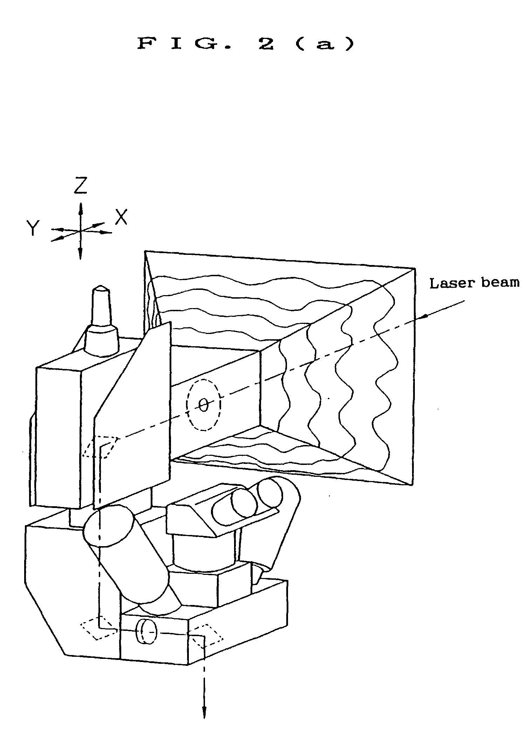

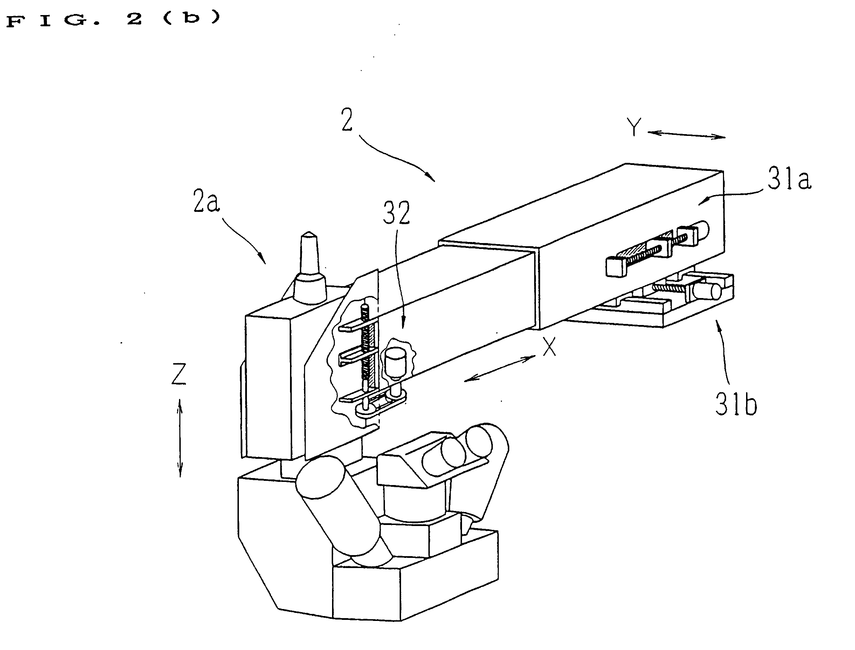

Fig. 1 is an external view showing a schematic construction of an apparatus for a corneal surgery by using an excimer laser beam. Reference numeral 1 indicates a primary body of a surgery apparatus, and the excimer laser beam source and the like are contained in the primary body 1. The laser beam irradiated from the excimer laser beam source is reflected by mirrors and is guided to an arm part 2. The inside of the arm part 2 is provided with a light course for a laser beam, and the optical elements, such as mirror and the like, are disposed in it (see Fig. 2(a)). The arm part 2 contains a microscope 3, an illumination part 4, and undermentioned eyeball position detecting optical system and so on, and moves in X-direction (right and left direction against the ophthalmologist) by being controlled by the X-direction arm driving device 31a and in Y-direction (before and behind direction against the ophthalmologist) by being controlled by the Y-direction arm driving device 31b, and an arm tip 2a moves in Z-direction (direction of the laser beam irradiating optical axis) by being controlled by the Z-direction arm driving device 32. Respective arm driving devices include motors and sliding mechanism (see Fig. 2(b)).

Reference numeral 6 indicates a controller including a joystick 7 for giving a signal to control the arm part 2 in XY-direction, and several kinds of operating switches. Reference numeral 8 indicates a foot switch for sending a laser output signal and reference numeral 9 indicates a computer for inputting some kinds of necessary data of operating condition, for calculating, displaying and storing the laser irradiating data, or the like. Reference numeral 10 is a bed for making the patient lie down thereon.

### [Constructions of Respective Components]

### (Optical system)

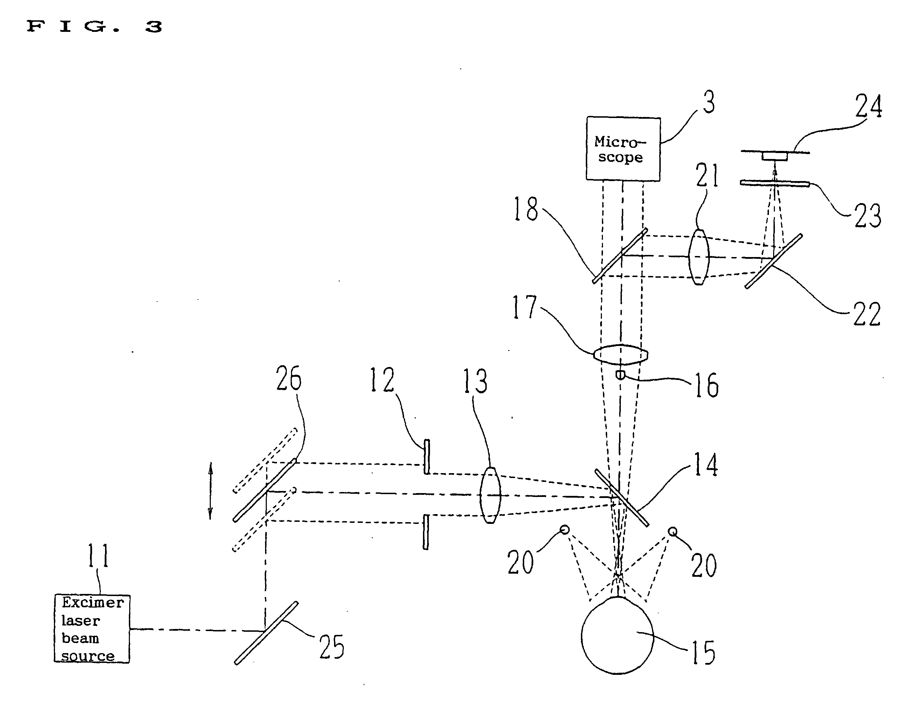

Fig. 3 is a schematic view showing the optical system of the apparatus.

### (A) A laser beam irradiating system

Reference numeral 11 indicates a laser beam source for irradiating an excimer laser beam of 193 nm wave length. The laser beam irradiated from the laser beam source 11 is guided to an arm part 2 by mirrors 25 and 26 or the like. Reference numeral 12 indicates an aperture for limiting an irradiating region of the laser beam, and of which the aperture diameter is changed by an aperture driving device. Reference numeral 13 is a projection lens for projecting an aperture 12 on a cornea of a patient eye 15. Through projection lens 13, the aperture 12 is located on conjugate position with the cornea, and the region limited by the aperture 12 is projected on the cornea, and then the region of keratectomy is limited. The laser beam having rectangular cross section irradiated from a laser beam source 11 moves to the predetermined direction based on the parallel movement of the mirror 26, and covers whole region of the aperture 12. These optical system is mentioned by Japanese Patent Publication (kokai) No.HEI4-242644(1992) (USP5, 507, 799) to which we want to be referred.

Reference numeral 14 indicates a dichroic mirror for reflecting an excimer laser beam and transmitting visible rays and infrared rays, and makes the optical axis of the laser beam irradiating optical system share the optical axis with the optical axis both of the observation system and the eyeball position detecting system, which will be undermentioned.

### (B) An observation system

Reference numeral 17 indicates an objective lens and reference numeral 18 indicates a dichroic mirror for transmitting visible rays and for reflecting.infrared rays. An image of anterior portion of the patient eye 15, which is illuminated by visible illumination light beam transmitted from the illumination part 4, is transmitted into the microscope 3 through the dichroic mirror 14, the objective lens 17 and the dichroic mirror 18. The ophthalmologist observes the patient eye 15 by using the binocular microscope 3. The reticle plate, which is not shown, is inserted into the observation system and the reticle plate can be the standard for the alignment in XY-direction of the patient eye.

Also, an object projecting system composed of two slits (referred to Japanese Publication (kokai) No.HEI6-47001(1994) (U.S. Patent application number 08/090,611 published as US-A-5 562 656 subsequent to the filing date of the present application)) is disposed in the observation optical system for alignment in Z-axis direction.
Reference numeral 16 indicates a fixation lamp placed on the observation optical axis.

### (C) An eyeball position detecting system

Reference numeral 20 indicates an IR-rays illumination light source such as LED or the like, and 4 members of IR-rays illumination light sources 20 are disposed at intervals of 90° each other around the optical axis as the center. Reference numeral 21 is an image camera lens, reference numeral 22 is a reflecting mirror, reference numeral 23 is an IR-rays transmitting filter, and reference numeral 24 is a CCD camera.

A flux of light beams of anterior portion image of the patient eye illuminated by the IR-rays illumination light source 20 is reflected by the dichroic mirror 18 through both the dichroic mirror 14 and objective lens 17. After that, the image is formed on the photo-imaging plain of the CCD camera 24 by an image camera lens 21 through both the reflecting mirror 22 and the IR-rays transmitting filter 23. At this moment, the IR-rays transmitting filter 23 cuts visible rays reflected slightly by the dichroic mirror 18.

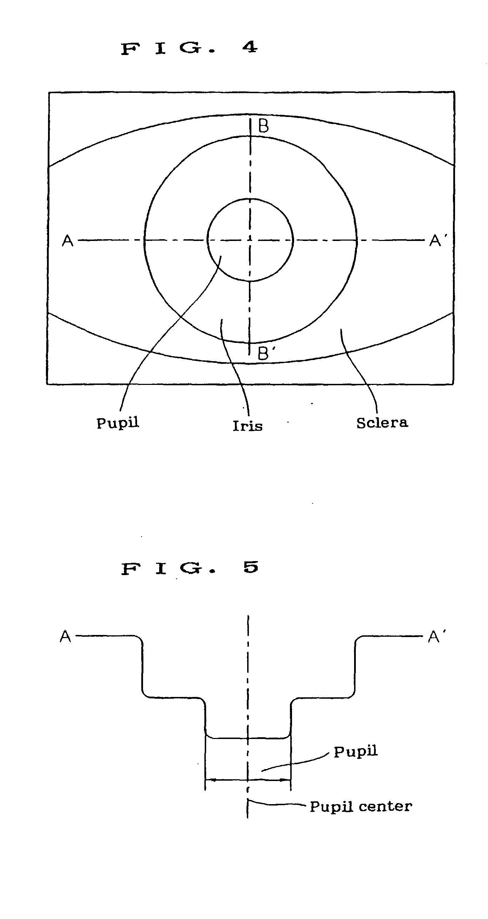

A CCD camera 24 detects the eyeball position as followings. Fig. 4 is a view of anterior portion image of the eye photographed by the CCD camera 24, and Fig. 5 is a view of distribution of light volume on line A-A' (Fig. 4) based on the image signal transmitted from the CCD camera 24. As designated in figures, the respective light volumes are different by which the pupil, the iris and the sclera, therefore according to the information, the pupil edge coordinates in lateral direction can be detected, furthermore, in accordance with the detection of the pupil edge, the center position, so called, the pupil center coordinates in lateral direction is can be obtained. In the same way as is described, the pupil center coordinates in longitudinal direction can be obtained by using information of distribution of light volume on longitudinal line B-B'. Therefore, in accordance with both of them, the pupil position against the optical axis of the detecting optical system (so called the optical axis of the laser beam irradiating optical system), which is regulated so as to be the predetermined positional relation on the photo-imaging elements of the CCD camera 24 is obtained. Still, the lateral and longitudinal detecting lines are desirable to be averaged in response to the information concerning distribution of light volume of plural lines which centers the middle of photo-imaging elements of the CCD camera 24. Furthermore, if the processing time permits, the center position of gravity is obtained based on the whole pupil region.

In addition, the reflecting light of the cornea transmitted from the IR-rays illumination light source 20 is also transmitted to the CCD camera 24, but by placing the IR-rays illumination light source 20 so that the reflecting image of the cornea may not interrupt the detecting lines (lateral and longitudinal), the reflecting light of the cornea is prohibited from being noise light in the case that the positional coordinates is detected. Namely, it makes the line, which linked by two opposite light sources, slant to 45° against the detecting lines (lateral and longitudinal), and makes the image of light source to be seen around the cornea. Besides, the composition of such arrangement as described above is not indispensable, in a word, it is satisfied by that the pupil edge (or an iris) can be detected.

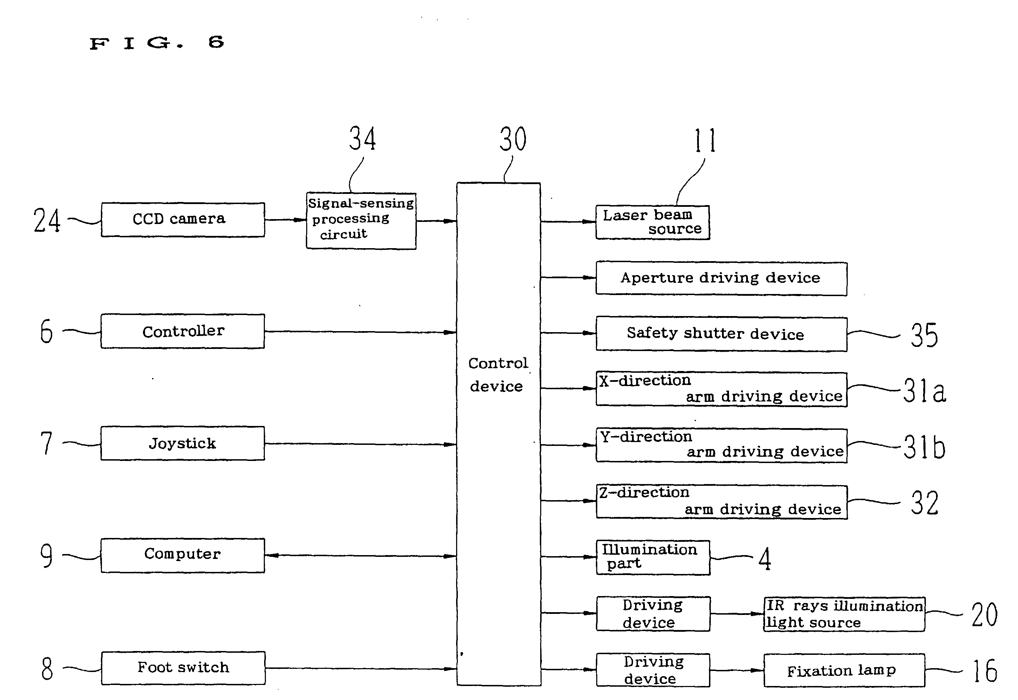

The operation of the apparatus, having such architecture as described above, will be described below with reference to the block diagram of the principal section of the control system shown in Fig. 6.

The ophthalmic surgery apparatus is connected to a power source and is made the system run, the menu frame is displayed at the CRT monitor display of the computer 9. The ways of the cornea operation by using an excimer laser beam are PRK (photorefractive keratectomy) operating mode and PTK (phototherapeutic keratectomy) operating mode, in this case, the ophthalmologist selects the PRK operating mode from the menu frame. The ophthalmologist inputs several kinds of data such as the refractive power value of the patient eye 15 and operating conditions or the like, which are detected beforehand, by using the keyboard of the computer 9. The computer 9 calculates the resection volume of the cornea or the like based on the inputted data. The calculated operating data are transmitted to the control device 30 instructed by keyboard operation.

After the input preparations have been completed, the ophthalmologist makes the patient lie down on the bed 10 and the ophthalmologist places the arm part 2 provided a laser beam irradiating orifice above the patient eye 15. The ophthalmologist turns on each of the light sources, and fixes the fixation lamp 16 to the patient eye 15.

The ophthalmologist observes anterior portion of the patient eye 15 illuminated by the illumination part 4 though the microscope 3, and he each aligns XY-direction by operating the joystick 7 and aligns Z-direction by operating the focus adjustment switch so as to be the predetermined relation between the reticle, which is not shown, and the pupil. After the signal generated by joystick 7 (and focus adjustment switch 60) has been inputted to the control device 30, the control device 30 makes the XY-direction arm driving devices 31a, 31b (and the Z-direction arm driving device 32) go into run, and moves the arm part 2 to XY-direction (and Z-direction).

During this alignment, if the automatic alignment changing switch 61, which is mounted on the controller 6, is turned on, the automatic alignment goes into run. In the case that the patient eye exists within the region where the pupil center position can be detected in the eyeball position detecting system, the apparatus makes the X, Y-direction arm driving devices 31a, 31b go into run so that the optical axis of the laser irradiating optical system can be coincide with the pupil center, and then makes the arm part 2 move to XY-direction.

In the case that the laser irradiation is performed on the condition that the optical axis of the laser beam irradiating optical system is coincide with the pupil center, after he has completed the alignment by turning on the automatic alignment changing switch, and then once he pushes the ready switch 62 mounted on the controller 6, the eyeball tracking mechanism goes into operation, which stores the predetermined position on the photo-imaging elements (the position of the optical axis of the laser beam irradiating optical system) of the CCD camera 24 as the standard position and moves the arm part 2 so that the pupil center can be coincide with the standard position.

The pupil center position obtained by processing signals of the CCD camera 24 is compared with the standard position at any time, if the patient eye 15 moves in outside of the predetermined permissible region, the control device 30 moves the arm part 2 to the XY-direction by driving the X,Y-direction arm driving devices 31a, 31b on the bases of the comparative information, and makes the center position of the pupil be within the permissible region of the standard position. Moreover, in the case that the patient eye moves in outside of the XY-direction moving region, the apparatus makes the safety shutter device 35 goes into run, and cuts off the laser beam irradiation.

After the eyeball tracking mechanism has run, the safety shutter 35 is released and the irradiation of the excimer laser beam is made ready. Then, after the ophthalmologist steps on the foot switch 8, the control device 30 emits the laser beam. The excimer laser beam is transmitted to the patient eye 15 through the laser beam irradiating optical system, and the cornea is ablated.

In the case that without using automatic alignment, the eyeball tracking is performed on the basis of the optical axis position of the laser beam irradiating optical system decided by the alignment operation by using the joystick, the optical axis of the laser beam irradiating optical system is placed on the target position by operating both the joystick 7 and the focus adjustment switch 60. After the alignment has been completed, and then, once the ready switch is pushed on, the pupil center position of the patient eye 15 is stored as the standard position (the standard position of this moment is different from the optical axis of the laser beam irradiating optical system). The eyeball tracking mechanism, which moves the arm part 2 so that the detected pupil position may coincide with the stored standard position, goes into run and the cornea is ablated the same as above mentioned.

In preferred embodiment as is described, the eyeball position detection calculates the pupil edge based on the information of distribution of light volume of anterior portion of the eye, and thereby the pupil center is obtained on the basis of the pupil edge, but, among the reflecting light volume of the pupil, that of iris, that of sclera and that of cillosis, the reflecting light volume transmitted from the pupil is extremely little, so it can be also capable of detecting the pupil position (displacement direction from the optical axis) according to the light-and-shade degree of light volume distribution on the photo-imaging plane of the CCD camera 24. In followings, the alignment performed by this detection will be described.

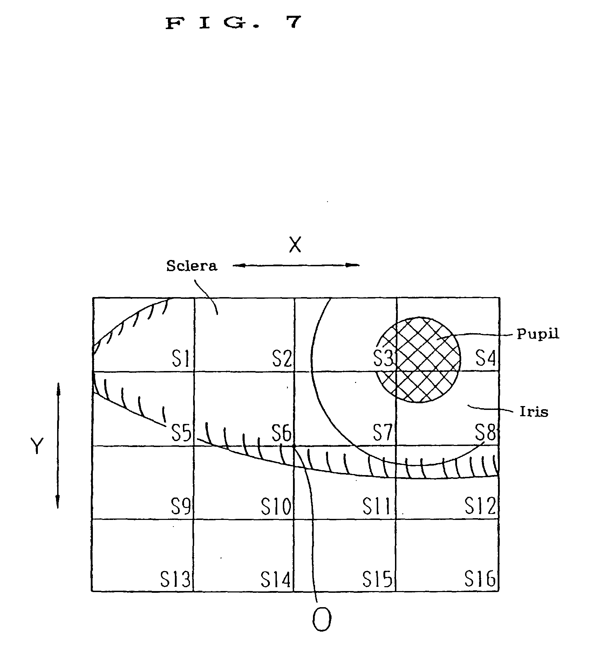

At first, on detecting the light-and-shade information of the reflecting light transmitted from anterior portion, as shown in Fig. 7, the image which centers the optical axis O is divided into 16 areas (S1 ∼ S16) of 4 × 4 against the two-dimensional image transmitted from the CCD camera 24. The positions of the pixel are previously determined, against the pixel of respective areas (125 pixel × 125 pixel), so that the predetermined number of pixel (for example, 64 articles), which are detecting objects for light-and-shade, may equally distributed in the area (all the pixel are can be the detecting objects, but if the required number for detecting are taken as the objects, the processing speed can be faster). The image signals transmitted from the CCD camera 24 are transformed into digital data by the signal-process sensing circuit 34, and then, processed by predetermined process, and inputted into the control device 30. Based on the inputted signals, the control device 30 obtains the light-and-shade degree in response to the pixel which are previously determined every area. Since the light-and-shade degree every one pixel is transformed into digital data, therefore, for example, it can be obtained as the numerical value of light-and-shade degree of 256 grades from 0 to 255 (0 side is the darkest, 255 side is the brightest).

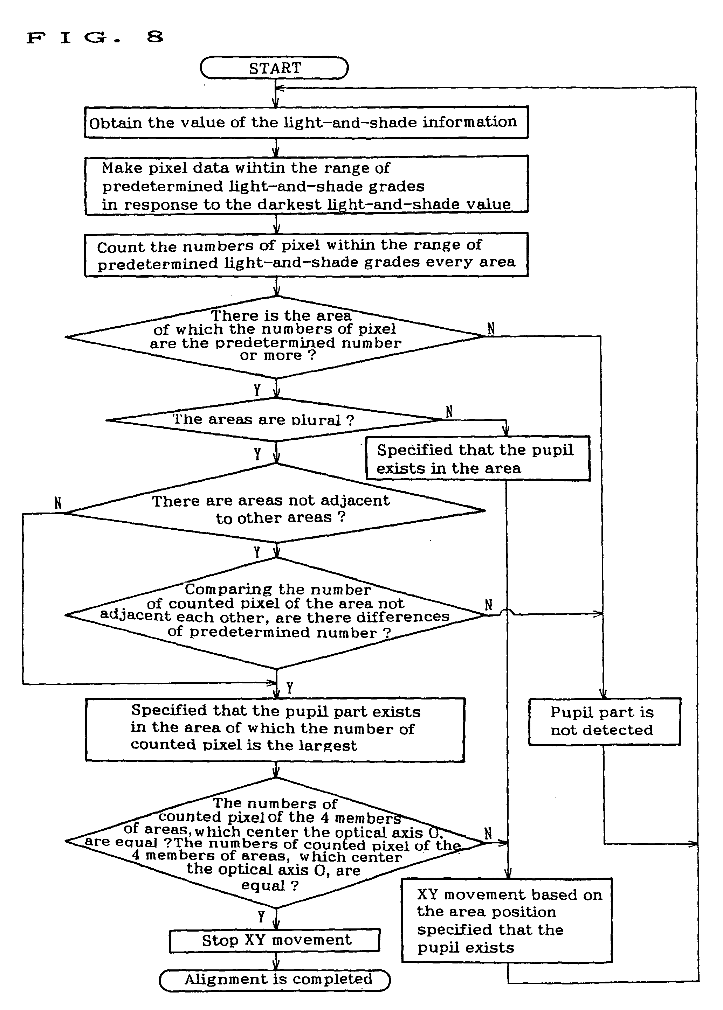

Then, the operation of the automatic alignment, which detects the pupil part based on the obtained light-and-shade value, and drives and controls the arm part 2 in XY-direction, will be described hereinafter with referring to the flowchart shown in Fig. 8.

If the automatic alignment changing switch 61 is turned on, the automatic alignment goes into run according to the pupil part detection based on the light-and-shade value. The control device 30 obtains the value of the light-and-shade information of the image which is previously determined every area, and then, takes out the light-and-shade information of predetermined grades (for example, 20 grades) based on the lowest (the darkest) grade of the light-and-shade value as the standard among them, and counts the number of pixel within the predetermined range of light-and-shade value in respective areas. Then, it is judged that whether the number of pixel, which are counted in respective areas, satisfies the predetermined number (for example, 20 articles) or more, or not. In the case that it satisfies the predetermined number, it is judged that the pupil part (or the iris part around the pupil) is in the area. In the case that the number of pixel is little, it is judged that the pupil part is not detected, since only a section of the pupil part is in the area, or the section, of which the light-and-shade value is low such as eyelashes, is overlapped on.

In the case that there are the plural areas which satisfy the predetermined number (20 articles) and there are ones which are not adjacent each other among the plural areas, it is judged that whether there are differences of predetermined number (10 articles) or not by comparing the number of counted pixel of the area having largest number with the number of counted pixel of the area not adjacent to the area having largest number. Because, even if there are eyelashes detached from the pupil part, in the case that there are some differences between the number of counted pixel, it is taken that the pupil part exists in the area which has larger number, and thereby it is distinguished from eyelashes or the like. In the case that there are not the differences of predetermined number (10 articles), it is taken that the pupil part is not detected. In the case that there are the differences of predetermined number, it is specified that the pupil part exists in the area of which the number of counted pixel are the largest, and after that, it is judged that whether the number of counted pixel of the 4 members of areas of S6, S7, S10, S11, which center the optical axis O, are equal (equal within the predetermined range) or not, if there are differences, according to the position of the area of which the pupil exists, X,Y-direction arm driving device 31a, 31b are driven and controlled toward the direction to which the differences are dissolved. And then, when the differences of number of counted pixel of the 4 members of areas of S6, S7, S10, S11 are dissolved completely, the drive toward X and Y-direction is made to stop (is not made to drive), thereby the automatic alignment has been completed.

Still, on detecting the pupil part on the basis of the above mentioned light-and-shade information, the division of the area in response to the two-dimensional image transmitted from the CCD camera 24, it can also be capable of dividing into 4 areas which center the optical axis O, simply.

Additionally, concerning the movement control of the arm part 2, it is more convenience to use together with the movement control based on the pupil center calculated by using the above mentioned pupil edge. The alignment in large range is performed according to the pupil part detection based on the light-and-shade information, and when the pupil edge detection is made possible, it is performed according to the pupil edge detection based on the pupil center position. Thereby, if it can be capable of observing the pupil part of the patient eye in the observation optical system, both the alignment in large range and the more accurate alignment are realized.

The present invention may be embodied in other specific forms. For instance, this invention can be carried out without involving the construction of the optical system of the laser beam irradiating optical system such as refraction reform or the like, and in the case of the eyeball tracking which based on the optical axis position, decided by the ophthalmologist, of the laser beam irradiating optical system, the alignment operation is also easier by deciding the position in previous inspection and inputting the difference between the position and the pupil center.

The forgoing description of the preferred embodiment of the invention has been presented for purposes of illustration and description. It is not intended to be exhaustive or to limit the variations to the precise form disclosed, and modifications and variations are possible in light of the above teachings or may be acquired from practice of the invention. The embodiment chosen and described in order to explain the principles of the invention and its practical application to enable one skilled in the art to utilize the invention in various embodiments and with various modifications as are suited to the particular use contemplated.

It is intended that the scope of the invention be defined by the claims appended hereto.

## Claims

1. An ophthalmic, surgery apparatus comprising:
an observation optical system (3, 14, 17, 18) for observing an anterior portion of an eye (15) of a patient; and
a laser beam irradiating optical system (11, 12, 13, 14, 25, 26) for producing an irradiating laser beam for causing ablation of a cornea of the eye,
means for aligning said irradiating optical system with the eye
said ophthalmic surgery apparatus being adapted to ablate the cornea of an area to be used for sight of the eye under the condition that said irradiating optical system is aligned with the eye, the apparatus comprising:
moving means (31 a, 31b) for moving an irradiating position of the laser beam by said irradiating optical system;
illuminating means (20) for illuminating an anterior portion of the eye of the patent including an iris of the eye;
photoelectric transducing means having a two-dimensional photographing element (24) for photographing a pupil and the iris of the eye illuminated by said illuminating means;
**characterized in that** the apparatus comprises:
pupil center position detecting means (30, 34) for detecting the position of a pupil center based on pupil information obtained by processing a signal transmitted from said photographing element;
storage means (30) for storing the pupil center position detected by said pupil center position detecting means when alignment before surgery is completed or a predetermined position of said photographing element; and
control means (30) for controlling said moving means by comparing the detected pupil center position and the stored position and obtaining deviation between the detected pupil center position and the stored position so that the detected pupil center position may be placed within a predetermined permissible range of the stored position.

2. An ophthalmic surgery apparatus according to claim 1, wherein said laser beam irradiating optical system includes a light source (11) for generating an excimer laser beam, whereby said excimer laser beam may be irradiated onto the cornea so that the cornea is ablated.

3. An ophthalmic surgery apparatus according to claim 1, wherein said control means comprises tracking signal generating means for generating a signal for driving said moving means so that the detected pupil center position may be placed within the predetermined permissible range of the stored position when the pupil center position is detected.

4. An ophthalmic surgery apparatus according to claim 1, comprising
irradiation prohibiting means (30, 35) for prohibiting irradiation of the laser beam when the detected pupil center position lies beyond the predetermined permissible range of the stored position.

5. An ophthalmic surgery apparatus according to claim 1, comprising
irradiation signal generating means (30) for generating a signal for permitting irradiation of the laser beam when the detected pupil center position is within the predetermined permissible range of the stored position.

6. An ophthalmic surgery apparatus according to claim 1, wherein said illuminating means includes an illumination light source which emits light in the near-infrared region and said photographing element has sensitivity to the near-infrared region, the illumination light source being disposed so that an image of the illumination light source is formed on a side of a periphery of the cornea relative to an edge of the pupil.

7. An ophthalmic surgery apparatus according to claim 1, wherein said pupil center position detecting means comprises arithmetic means for calculating the position of an outer circumference of the pupil based on the pupil information and calculating the pupil center position based on the position of the outer circumference of the pupil.

8. An ophthalmic surgery apparatus according to claim 1, comprising:
light-and-shade sensing means (30, 34) for detecting pixels of said photographing element in a plurality of divided regions and then obtaining light-and-shade information of each said divided region; and
determining means (30) for determining a moving direction of the irradiation position by comparison of a distribution condition among each divided region having a distribution of pixels of a predetermined low photo-volume level,
wherein said control means controls said moving means based on the determined moving direction.

9. An ophthalmic surgery apparatus according to claim 1, wherein said observation optical system has an optical axis which coincides with an optical axis of said laser beam irradiating optical system, and said moving means also moves said observation optical system.

## Patentansprüche

1. Gerät für die Augenchirurgie mit:
- einem optischen Beobachtungssystem (3, 14, 17, 18) zur Beobachtung eines vorderen Abschnitts eines Auges (15) eines Patienten; und
- einem optischen Laserstrahl-Bestrahlungssystem (11, 12, 13, 14, 25, 26) zur Erzeugung eines bestrahlenden Laserstrahls, um eine Hornhautabtragung zu bewirken;
- einem Mittel zur Ausrichtung des optischen Bestrahlungssystems mit dem Auge;
- wobei das Gerät für die Augenchirurgie ausgelegt ist, um die Hornhaut in einem Bereich abzutragen, der für die Sicht des Auges verwendet wird, sofern das optische Bestrahlungssystem mit dem Auge ausgerichtet ist, wobei das Gerät umfasst:
- ein Bewegungsmittel (31a, 31b) zur Bewegung einer Bestrahlungsposition des Laserstrahls von dem optischen Bestrahlungssystem;
- ein Beleuchtungsmittel (20) zur Beleuchtung eines vorderen Abschnitts des Auges des Patienten, der die Iris des Auges enthält;
- ein fotoelektrisches Umwandlungsmittel, das ein zweidimensionales fotografisches Element (24) zum Fotografieren einer Pupille und der Iris des mit dem Beleuchtungsmittel beleuchteten Auges umfasst;
**dadurch gekennzeichnet, dass** das Gerät umfasst:
- ein Pupillenzentrum-Positionserfassungsmittel (30, 34) zur Erfassung der Position eines Pupillenzentrums auf der Grundlage einer Pupulleninformation, die durch Verarbeitung eines von dem fotografischen Element übertragenen Signals gewonnen wird;
- ein Speicherungsmittel (30) zur Speicherung der von dem Pupillenzentrum-Positionserfassungsmittel erfassten Position des Pupillenzentrums, sobald eine Ausrichtung vor der Operation abgeschlossen ist, oder einer vorbestimmten Position des fotografischen Elements; und
- ein Steuerungsmittel (30) zur Steuerung des Bewegungsmittels durch Vergleichen der erfassten Position des Pupillenzentrums und der gespeicherten Position, und zur Gewinnung einer Abweichung zwischen der erfassten Position des Pupillenzentrums und der gespeicherten Position, so dass die erfasste Position des Pupillenzentrums innerhalb eines vorbestimmten erlaubten Bereichs der gespeicherten Position angeordnet werden kann.

2. Gerät für die Augenchirurgie nach Anspruch 1, wobei das optische Laserstrahl-Bestrahlungssystem eine Lichtquelle (11) zur Erzeugung eines Excimerlaserstrahls umfasst, wobei der Excimerlaserstrahl auf die Hornhaut gestrahlt werden kann, so dass die Hornhaut abgetragen wird.

3. Gerät für die Augenchirurgie nach Anspruch 1, wobei das Steuerungsmittel ein Nachführsignal-Erzeugungsmittel zur Erzeugung eines Signals zur Ansteuerung des Bewegungsmittels umfasst, so dass die erfasste Position des Pupillenzentrums innerhalb des vorbestimmten erlaubten Bereichs der gespeicherten Position angeordnet werden kann, wenn die Position des Pupillenzentrums erfasst ist.

4. Gerät für die Augenchirurgie nach Anspruch 1, das ein Bestrahlungsverhinderungsmittel (30, 35) umfasst, um eine Bestrahlung durch den Laserstrahl zu verhindern, wenn die erfasste Position des Pupillenzentrums außerhalb des vorbestimmten erlaubten Bereichs der gespeicherten Position liegt.

5. Gerät für die Augenchirurgie nach Anspruch 1, das ein Bestrahlungssignal-Erzeugungsmittel (30) umfasst, um ein Signal zu erzeugen, so dass eine Bestrahlung durch den Laserstrahl erlaubt ist, wenn die erfasste Position des Pupillenzentrums innerhalb des vorbestimmten erlaubten Bereichs der gespeicherten Position liegt.

6. Gerät für die Augenchirurgie nach Anspruch 1, wobei das Beleuchtungsmittel eine Beleuchtungslichtquelle umfasst, die Licht im nahen Infrarotbereich aussendet, und das fotografische Element eine Empfindlichkeit im nahen Infrarotbereich aufweist, wobei die Beleuchtungslichtquelle so angeordnet ist, dass ein Bild der Beleuchtungslichtquelle relativ zu einem Rand der Pupille in einem äußeren Bereich der Hornhaut erzeugt wird.

7. Gerät für die Augenchirurgie nach Anspruch 1, wobei das Pupillenzentrum-Positionserfassungsmittel ein arithmetisches Mittel zur Berechnung der Position eines äußeren Umfangs der Pupille auf der Grundlage der Pupilleninformation und zur Berechnung der Position des Pupillenzentrums auf der Grundlage der Position des äußeren Umfangs der Pupille umfasst.

8. Gerät für die Augenchirurgie nach Anspruch 1, mit:
- einem Licht-und-Schatten-Erfassungsmittel (30, 34) zur Erfassung von Pixeln des fotografischen Elements in einer Mehrzahl von unterteilten Bereichen und zur anschließenden Gewinnung einer Licht-und-Schatten-Information von jedem der unterteilten Bereiche; und
- einem Bestimmungsmittel (30) zur Bestimmung einer Bewegungsrichtung der Bestrahlungsposition, indem ein Verteilungszustand aller unterteilten Bereiche verglichen wird, die eine Verteilung von Pixeln mit einer vorbestimmten geringen Helligkeit aufweisen;
- wobei das Steuerungsmittel das Bewegungsmittel auf der Grundlage der vorbestimmten Bewegungsrichtung steuert.

9. Gerät für die Augenchirurgie nach Anspruch 1, wobei das optische Beobachtungssystem eine optische Achse aufweist, die mit einer optischen Achse des optischen Laserstrahl-Bestrahlungssystems zusammenfällt, und das Bewegungsmittel ebenfalls das optische Beobachtungssystem bewegt.

## Revendications

1. Appareil de chirurgie ophtalmique, comprenant :
un système optique d'observation (3, 14, 17, 18) destiné à l'observation d'une partie antérieure d'un oeil (15) d'un patient, et
un système optique (11, 12, 13, 14, 25, 26) d'irradiation par un faisceau laser destiné à produire un faisceau laser d'irradiation destiné à provoquer l'ablation d'une cornée de l'oeil,
un dispositif d'alignement du système optique d'irradiation sur l'oeil,
l'appareil de chirurgie ophtalmique étant destiné à assurer l'ablation de la cornée dans une région destinée à être utilisée pour la visée de l'oeil dans des conditions dans lesquelles le système optique d'irradiation est aligné sur l'oeil, l'appareil comprenant :
un dispositif de déplacement (31a, 31b) destiné à déplacer une position d' irradiation du faisceau laser par le système optique d'irradiation,
un dispositif d'éclairage (20) destiné à éclairer une partie antérieure de l'oeil du patient comprenant un iris de l'oeil, et
un dispositif transducteur photoélectrique ayant un élément photographique bidimensionnel (24) destiné à photographier une pupille et l'iris de l'oeil éclairé par le dispositif d'éclairage,
**caractérisé en ce que** l'appareil comprend :
un dispositif (30, 34) de détection de position centrale de la pupille destiné à détecter la position d'un centre de pupille d'après des informations de pupille obtenues par traitement d'un signal transmis par l'élément photographique,
un dispositif (30) de mémorisation destiné à conserver la position centrale de la pupille détectée par le dispositif de détection de position centrale de pupille lors de l'alignement avant chirurgie et réalisé à une position prédéterminée de l'élément photographique, et
un dispositif de commande (30) destiné à commander le dispositif de déplacement par comparaison de la position détectée du centre de la pupille et de la position mémorisée et à obtenir l'écart entre la position détectée du centre de la pupille et la position mémorisée afin que la position détectée du centre de la pupille puisse être placée dans une plage permise prédéterminée de la position mémorisée.

2. Appareil de chirurgie ophtalmique selon la revendication 1, dans lequel le système optique d'irradiation par un faisceau laser comporte une source de lumière (11) destinée à créer un faisceau de laser à excimère, si bien que le faisceau du laser à excimère peut être projeté sur la cornée et la cornée peut subir l'ablation.

3. Appareil de chirurgie ophtalmique selon la revendication 1, dans lequel le dispositif de commande comprend un dispositif générateur d'un signal de poursuite destiné à créer un signal de pilotage du dispositif de déplacement afin que la position détectée du centre de la pupille puisse être placée dans une plage permise prédéterminée de la position mémorisée lorsque la position du centre de la pupille est détectée.

4. Appareil de chirurgie ophtalmique selon la revendication 1, comprenant :
un dispositif (30, 35) destiné à empêcher l'irradiation qui est destinée à empêcher la projection du faisceau laser lorsque la position détectée du centre de la pupille est au-delà de la plage permise prédéterminée de la position mémorisée.

5. Appareil de chirurgie ophtalmique selon la revendication 1, comprenant
un dispositif (30) générateur d'un signal d'irradiation destiné à créer un signal destiné à permettre la projection du faisceau laser lorsque la position détectée du centre de la pupille se trouve dans la plage permise prédéterminée de la position mémorisée.

6. Appareil de chirurgie ophtalmique selon la revendication 1, dans lequel le dispositif d'éclairage comprend une source de lumière d'éclairage qui émet de la lumière dans la région du proche infrarouge, et l'élément photographique a une sensibilité dans la région du proche infrarouge, la source de lumière d'éclairage étant disposée de manière qu'une image de la source de lumière d'éclairage soit formée d'un côté d'une périphérie de la cornée par rapport à un bord de la pupille.

7. Appareil de chirurgie ophtalmique selon la revendication 1, dans lequel le dispositif de détection de la position du centre de la pupille comprend un dispositif arithmétique destiné à calculer la position d'une circonférence externe de la pupille d'après des informations de pupille et à calculer la position du centre de la pupille d'après la position de la circonférence externe de la pupille.

8. Appareil de chirurgie ophtalmique selon la revendication 1, comprenant :
un dispositif (30, 34) de détection d'ombre et de lumière destiné à détecter des éléments d'image de l'élément photographique dans plusieurs régions divisées, puis à obtenir des informations d'ombre et de lumière de chaque région divisée, et
un dispositif (30) de détermination d'une direction de déplacement de la position d'irradiation par comparaison d'une condition de distribution parmi chaque région divisée ayant une distribution d'éléments d'image de faible niveau prédéterminé de volume photographique,
dans lequel le dispositif de commande assure la commande de dispositif de déplacement d'après la direction déterminée de déplacement.

9. Appareil de chirurgie ophtalmique selon la revendication 1, dans lequel le système optique d'observation a un axe optique qui coïncide avec un axe optique du système optique d'irradiation par un faisceau laser, et le dispositif de déplacement déplace aussi le système optique d'observation.
